# EUROPÄISCHE PATENTANMELDUNG

(11) **EP 0 943 912 A1**
(43) Veröffentlichungstag der Anmeldung: **22.09.1999**
(21) Anmeldenummer: 99104138.5
(22) Anmeldetag: 02.03.1999
(51) Int. Cl.: G01N 21/35, G01N 33/34

(54) **Einrichtung zum Nachweis von Wasser und wasserhaltigen Substanzen, insbesondere von wasserhaltigen Klebstoffen, auf Oberflächen beliebiger Materialien**

(30) Priorität: 05.03.1998 DE 19810163
(71) Anmelder: Valco Cincinnati GmbH, 32130 Enger (DE)
(72) Erfinder: Fink, Frank Prof.Dr., 10439 Berlin (DE)
(74) Vertreter: Specht, Peter, Dipl.-Phys.

(57) **Zusammenfassung**

Eine Einrichtung zum Nachweis von Wasser und wasserlöslichen Substanzen, insbesondere wasserhaltigem Klebstoff, auf Oberflächen beliebiger Materialien, insbesondere auf Klebeflächen aus Pappe und Papier beliebiger Farbe für die Verpackungsindustrie besteht aus einer Beleuchtungseinrichtung und zwei Detektoren. Mit der Beleuchtungseinrichtung wird Licht, dessen Spektrum die Absorptionsbande des Wassers bei 1,4µm und deren spektrale Umgebung überdeckt, auf die Oberfläche abgebildet. Das von der Oberfläche zurückgesandte Licht wird über eine Optik auf den Detektor, dem ein Spektralfilter vorgeschaltet ist, das Licht bei der 1,4pm-Absorptionsbande durchläßt, abgebildet. Ein Teil des von der Oberfläche zurückgesandten Lichtes wird über einen Strahlteiler auf einen zweiten Detektor geleitet, dem ein Spektralfilter vorgeschaltet ist, das Licht außerhalb der 1,4µm Absorptionsbande durchläßt.

Beide Signale werden einem Differenzverstärker zugeführt. Der Unterschied im Differenzsignal, der sich aus der Meßsituation Oberfläche mit wasserlöslicher Substanz zur Oberfläche ohne ergibt, wird als Indikator für das Vorhandensein der wasserlöslichen Substanz verwendet.

## Beschreibung

Die Erfindung betrifft eine Einrichtung zum Nachweis von Wasser und wasserhaltigen Substanzen, insbesondere wasserhaltigen Klebstoffen, auf verschiedenen Materialien mit unterschiedlicher Oberflächenbeschaffenheit und mit unterschiedlichen Farben, die vorzugsweise in der Verpackungsindustrie Anwendung findet.

Im Fertigungsprozeß von Verpackungen, vorzugsweise aus Papier und Pappe, wird der ortsgenaue Auftrag von wasserhaltigen Klebstoffen auf die dafür vorgesehenen Klebeflächen der Verpackungszuschnitte gefordert. Der Klebstoffauftrag erfolgt über gesteuerte Beleimungsdüsen, an denen die Klebeflächen mit hoher Geschwindigkeil (bis zu 10m/s) vorbeigeführt werden. Durch Störungen beim Auftragsprozeß kommt es vor, daß Klebeflächen falsch oder gar nicht beleimt werden. Um diese Verpackungszuschnitte rechtzeitig zu erkennen und steuernd in den Prozeß eingreifen zu können, werden Detektoren benötigt, die den Klebstoffauftrag mit der erforderlichen Geschwindigkeit kontrollieren.

Die bekannten Detektoren zum Nachweis des Klebstoffauftrags nutzen die Fluoreszenz von Substanzen aus, die dem Klebstoff vorab beigemischt und durch geeignete elektromagnetische Strahlung angeregt werden. Die Beimischung dieser fluoreszierenden Substanzen zum Klebstoff ist bei Verpackungen, die zum Beispiel für die Lebensmittelindustrie benötigt werden, unerwünscht.

Darüber hinaus können durch die elektromagnetische Strahlung auch die zu beleimenden Flächen selbst zur Fluoreszenz angeregt werden. Dies tritt insbesondere bei farbigen oder beschichteten Klebeflächen ein. Diese zusätzliche Fluoreszenz kann sich als Störfluoreszenz der Fluoreszenz der Klebstoffspur überlagern und zu Fehlinformationen führen.

Es ist Aufgabe der Erfindung, eine Einrichtung zum Nachweis von wasserhaltigen Substanzen, insbesondere von wasserlöslichen Klebstoffen der Verpackungsindustrie, anzugeben, die darüber hinaus den Klebstoff ohne Beimischung zusätzlicher Substanzen nachweist und unabhängig von der Farbe und der Oberflächenbeschaffenheit der Materialien, auf die die wasserlösliche Substanz oder der Klebstoff aufgetragen ist, arbeitet.

Diese Aufgabe wird erfindungsgemäß dadurch gelöst, daß über eine Optik ein Lichtfleck auf die Klebefläche mit der Klebstoffspur geleitet wird, dessen Spektrum die Absorptionsbande des Wassers bei 1,4µm überdeckt, und daß das von der Klebefläche und der Klebstoffspur zurückgeworfene Licht auf einen Photoempfänger für Infrarotstrahlung geleitet wird, vor dem ein Filter angeordnet ist, das nur Licht bei der 1,4µm-Absorptionsbande des Wassers durchläßt. Das von der Klebefläche ohne Klebstoffspur empfangene Signal unterscheidet sich deutlich von dem Signal, das von einer Klebefläche mit Klebstoffspur empfangen wird. Die Spur mit dem wasserlöslichen Klebstoff schwächt durch Absorption im Bereich der Wasserbande um 1,4µm das auftretende Licht, so daß auch das zurückgeworfene Licht im Bereich der Absorptionsbande um 1,4µm geschwächt erscheint. Der Unterschied zwischen der mit dem Photoempfänger registrierten Lichtmenge, die von der Klebefläche ohne und mit Klebstoffspur entsteht, dient zur Erkennung der Klebstoffspur.

Für Materialien, die im Bereich der Absorptionsbande ebenfalls eine starke Absorption zeigen, wie z. B. farbige und schwarze Klebeflächen, wird bei einer zweiten Ausführungsform der Erfindung ein zweiter Detektorkanal eingerichtet, der Licht aus der unmittelbaren spektralen Umgebung der Absorptionsbande registriert. Dazu wird vor dem Photoempfänger ein entsprechendes Filter angeordnet, das Licht aus der spektralen Umgebung (z.B. um 1,3µm) der 1,4µm-Absorptionsbande, nicht jedoch bei der 1,4µm-Absorptionsbande selbst, durchläßt. Das so gewonnene Referenzsignal wird in einer nachgeschalteten Differenzbildungseinheit mit dem Signal, das im spektralen Bereich der Absorptionsbande ermittelt wurde, ausgewertet. Dadurch wird der Betrag der Klebefläche zur Absorption gegenüber dem Betrag von Klebefläche plus Klebstoff zur Absorption berücksichtigt. Das Differenzsignal dient erfindungsgemäß zur Erkennung der Klebstoffspur auf Oberflächen, die im Bereich der Wasserbanden störende eigene Absorptionseigenschaften zeigen.

Neben der Absorptionsbande bei 1,4µm läßt sich die oben beschriebene Anordnung erfindungsgemäß auch bei den Absorptionsbanden um 0,95µm, 1,7µm, 1,94µm oder 2,94µm betreiben. Dazu müssen die spektralen Eigenschaften des Beleuchtungslichts und der Filter von den Photoempfängern auf die jeweilige Absorptionsbande bzw. deren spektraler Umgebung angepaßt werden.

In einer weiteren Ausführungsform wird beim Betreiben der Einrichtung bei der Absorptionsbande um 1,4µm das Referenzsignal aus dem Spektralbereich unterhalb von 1,2µm gewonnen.

Die erfindungsgemäße Einrichtung ist in einer weiteren Ausführungsform dadurch gekennzeichnet, daß zur spektralen Trennung der Lichtanteile ein Gitter, vorzugsweise ein abbildendes Gitter, verwendet wird. Mit dem abbildenden Gitter werden die spektral aufgetrennten Lichtteile direkt auf die Photoempfänger geleitet.

Die mit der Erfindung erzielten Vorteile bestehen insbesondere darin, daß die wasserlösliche Substanz ohne den Zusatz von Fluoreszenzmarken auf sämtlichen Oberflächen, unabhängig von ihrer Farbe und Beschaffenheit, nachgewiesen werden kann.

Eine vorteilhafte Ausgestaltung der Erfindung ist im Patentanspruch 6 angegeben. Die Verwendung eines abbildenden Gitters vermeidet den Einsatz der teuren Spektralfilter und ersetzt die Abbildungsoptik vor den Photoempfängern, so daß eine kostengünstige Variante entsteht.

Durch die Verwendung von Meßstrahlung im infraroten Spektralbereich und der entsprechenden Filter ist die Einrichtung unempfindlich gegenüber Strahlung im sichtbaren Spektralbereich.

Ein Ausführungsbeispiel der Erfindung ist in der Zeichnung dargestellt und wird im folgenden näher beschrieben.

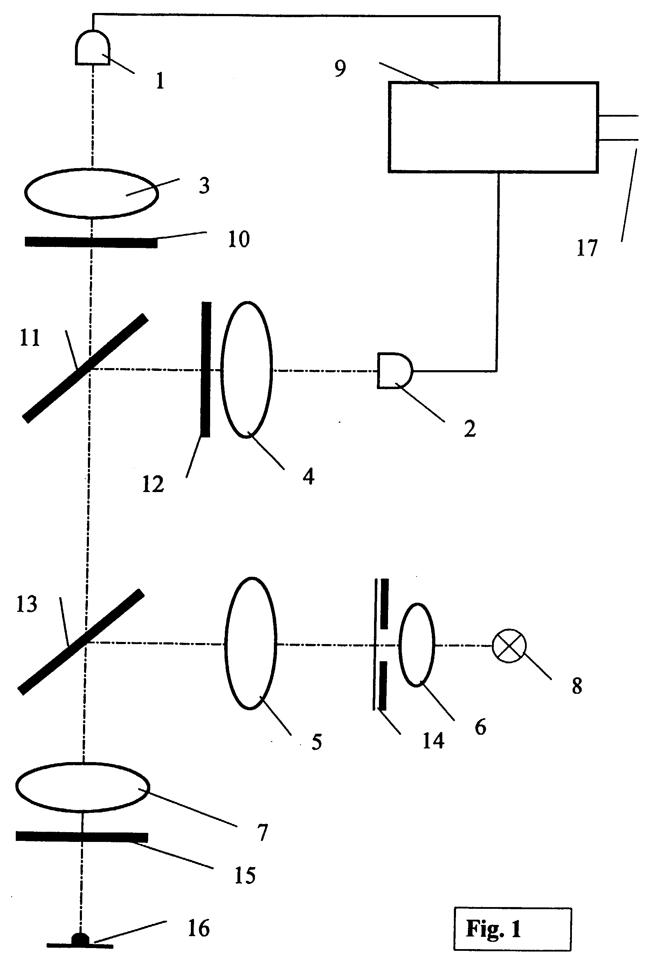

Es zeigt Fig.1 eine schematische Darstellung der Gesamtanordnung.

Die erfindungsgemäße Einrichtung weist eine Abbildungsoptik bestehend aus einer Bikonvexlinse (5) auf, die im Abstand der einfachen Brennweite vor einer Blende mit Streuscheibe (14) angeordnet ist. Die Blende mit Streuscheibe wird von der Glühlampe (8) über die Linse (6) beleuchtet. Das hinter der Linse (5) vorliegende parallele Lichtbündel trifft auf die Umlenkplatte (13). Über die Linse (7) wird der Lichtfleck, dessen Form durch die Blende (14) und den Abbildungsmaßstab der Optik bestimmt wird, auf die Probe (16) abgebildet. Das Kantenfilter (15) läßt nur Licht aus dem Spektralbereich durch, der die Wasserabsorptionsbande und die Referenzstrahlung überdeckt.

Das von der Probe zurückgesandte Licht wird nach dem Passieren des Filters (15) von der Linse (7) aufgenommen und als paralleles Lichtbündel durch die Umlenkplatte (13) hindurch auf den Teilerspiegel (11) gelenkt. Der Teilerspiegel wirkt spektral selektiv. Er reflektiert den Anteil des Lichtes, der außerhalb der Wasserabsorptionsbande (im Ausführungsbeispiel um 1,3µm) liegt und läßt den spektalen Lichtanteil, der bei der Wasserabsorptionsbande ( um 1,4µm) liegt, durch. Das durch den Teilerspiegel (11) tretende Licht wird durch das Filter (10) geleitet und mittels der Linse (3) auf die Photodiode (1) geschickt. Der vom Teilerspiegel (11) reflektierte Lichtteil gelangt durch das Filter (12) auf die Linse (4), die ihn auf die Photodiode (2) fokussiert.

Das Filter (10) läßt Licht bei der Wasserabsorptionsbande durch und das Filter (12) Licht aus der Umgebung der Absorptionsbande, im Ausführungsbeispiel um 1,3µm.

Die Signale der Photodioden (1) und (2) werden einem Differenzverstärker (9) zugeführt. Das Differenzsignal wird über die Anschlüsse (17) zu Prozeßsteuerung weitergeführt.

## Patentansprüche

1. Einrichtung zum Nachweis von Wasser und wasserlöslichen Substanzen, insbesondere wasserhaltigen Klebstoffen, auf Oberflächen beliebiger Materialien, insbesondere auf Klebeflächen aus Pappe und Papier beliebiger Farbe und Oberflächenbeschaffenheit sowie vor verschiedenen Hintergründen und Umgebungslichtbedingungen bestehend aus einer Beleuchtungseinrichtung, die elektromagnetische Strahlung auf die Oberfläche aussendet und einem Empfangsteil für die zurückgesandte Strahlung mit nachgeschalteter Auswerteeinheit **dadurch gekennzeichnet, daß** die Beleuchtungseinrichtung elektromagnetische Strahlung aussendet, die eine der Absorptionsbanden des Wassers oder des wasserhaltigen Klebstoffs im infraroten Spektralgebiet, vorzugsweise bei 1,4µm, und ihre spektrale Umgebung überdeckt, und daß der Empfangsteil aus einem infrarotempfindlichen Photoempfänger mit mindestens einem vorgeschalteten Filter besteht, das elektromagnetische Strahlung im Bereich der Absorptionsbande durchläßt.

2. Einrichtung nach Anspruch 1, **dadurch gekennzeichnet, daß** ein zweiter Empfangsteil verwendet wird, der neben dem Photoempfänger mindestens einen Filter enthält, das Strahlung in der Umgebung der Absorptionsbande durchläßt, diese jedoch im Bereich der Absorptionsbande selbst abblockt.

3. Einrichtung nach Anspruch 1 und 2, **dadurch gekennzeichnet, daß** die Signale der beiden Empfangskanäle einem Differenzverstärker zugeführt werden und das entstehende Differenzsignal zur Anzeige gebracht wird.

4. Einrichtung nach Anspruch 1 und 2, **dadurch gekennzeichnet, daß** die Signale der beiden Empfangskanäle einem Ouotientenbildner zugeführt werden und das entstehende Signal zur Anzeige gebracht wird.

5. Einrichtung nach Anspruch 1,2 und 3 oder 4, **dadurch gekennzeichnet, daß** für beide Empfangskanäle ein gemeinsamer wellenlängenselektiver Spiegel verwendet wird, der Strahlung im Bereich der 1,4µm Absorptionsbande durchläßt, wobei diese Strahlung auf den einen Photoempfänger gelangt und Strahlung außerhalb dieser Absorptionsbande reflektiert, wobei diese dem zweiten Photoempfänger zugeführt wird.

6. Einrichtung nach Anspruch 1 und 2, **dadurch gekennzeichnet, daß** das von dem Objekt zurückgesandte Licht spektral durch ein Gitter zerlegt wird und daß der spektrale Bereich der Strahlung um die 1,4µm-Absorptionsbande dem einen Photoempfänger und Strahlung außerhalb der jeweiligen Absorptionsbande dem anderen Empfänger zugeleitet wird.

7. Einrichtung nach Anspruch 1 bis 6, **dadurch gekennzeichnet, daß** anstelle der 1,4µm-Absorptionsbande eine der anderen Absorptionsbanden des Wassers oder des wasserhaltigen Klebstoffes bei 0,95pm, 1,7µm oder 2,9µm genutzt werden.

8. Einrichtung nach Anspruch 1 bis 7 **dadurch gekennzeichnet, daß** für die Strahlung außerhalb der Absorptionsbande Strahlung verwendet wird, die im spektralen Empfindlichkeitsbereich von Photoempfängern auf Siliziumbasis liegt.

9. Einrichtung nach Anspruch 1 bis 6, **dadurch gekennzeichnet, daß** anstelle der 1,4µm-Absorptionsbande die Absorptionsbande des Wassers oder des wasserhaltigen Klebstoffes bei 1,94µm genutzt wird.
